# EUROPEAN PATENT APPLICATION

(11) **EP 2 148 179 A1**
(43) Date of publication of application: **27.01.2010**
(21) Application number: 09009339.4
(22) Date of filing: 17.07.2009
(51) Int. Cl.: G01G 19/44, G01G 3/14, A61B 5/024, A61B 5/11, G01L 1/22, G01P 15/12

(54) **Load converter and measuring device**

(30) Priority: 25.07.2008 JP 2008191919
(71) Applicant: TANITA CORPORATION, Tokyo 174-8630 (JP)
(72) Inventor: Kodama, Masato, tokyo 174-8630 (JP)
(74) Representative: Müller-Boré & Partner Patentanwälte

(57) **Abstract**

According to one aspect of the invention, a load converter that converts a load into an electric signal, the load converter includes: a flexure element that is distorted in accordance with a load applied to the flexure element; a first strain sensor that is disposed on the flexure element to output a first electric signal corresponding to the distortion of the flexure element; and a second strain sensor that is disposed on the flexure element to output a second electric signal corresponding to the distortion of the flexure element, wherein the first strain sensor is more excellent in accuracy than the second strain sensor, and the second strain sensor is more excellent in responsiveness than the first strain sensor.

## Description

### BACKGROUND

### 1. Field

The present invention relates to a measuring technique using a strain sensor such as a strain gauge.

### 2. Description of the Related Art

An ordinary weight scale is provided with a load converter having a strain gauge stuck to a flexure element as disclosed in JP-A-2006-313096. As the strain gauge, a metal strain gauge is ordinarily used that is more excellent in accuracy than a semiconductor strain gauge. On the other hand, there is a measuring device that measures active information showing the living activity of a human being. As the measuring device of this kind, a measuring device having a strain gauge is well-known (see JP-A-2005-177471). As this strain gauge, the semiconductor strain gauge is ordinarily used that is more excellent in responsiveness than the metal strain gauge.

As described above, since the metal strain gauge is inferior in its responsiveness and the semiconductor strain gauge is inferior in its accuracy, a usual measuring device having only either of these strain gauges cannot measure both the weight of a living being such as the human being and the active information (for instance, the number of heart-beats) showing the living activity of the living being such as the human being with high performance.

The present invention is devised by considering such circumstances and it is an object of the present invention to provide a load converter and a measuring device that can measure both the weight and active information of a living being such as a human being with high performance.

### SUMMARY OF THE INVENTION

In order to solve the above-described problem, the present invention provides a load converter that converts a load into an electric signal. The load converter comprises:
a flexure element that is distorted in accordance with a transmitted load; a first strain sensor that is stuck to the flexure element to output the electric signal corresponding to the distortion of the flexure element; and a second strain sensor that is stuck to the flexure element to output the electric signal corresponding to the distortion of the flexure element, and is **characterized in that** the first strain sensor is more excellent in accuracy than the second strain sensor and the second strain sensor is more excellent in responsiveness than the first strain sensor. Since the load converter includes the first strain sensor excellent in its accuracy and the second strain sensor excellent in its responsiveness, the load converter is used so that both the weight of a living being such as a human being and active information showing the living activity of the living being can be measured with high performance.

In the above-described load converter, the first strain sensor is preferably a metal strain gauge and the second strain sensor is preferably a semiconductor strain gauge. In ordinary and various kinds of strain sensors, the metal strain gauge is especially excellent in its accuracy and the semiconductor strain gauge is especially excellent in its responsiveness. Further, the semiconductor strain sensor has a higher sensitively than those of other strain sensor (for instance, a piezoelectric element) especially excellent in their responsiveness. Accordingly, this load converter is used so that the weight and the active information can be measured with extremely high performance.

In the present invention, the word of accuracy means comprehensive excellence of trueness and precision.

Further, in order to solve the above-described problem, the present invention provides a measuring device that measures the weight of a living being and active information showing the living activity of the living being. The measuring device comprises: the above-described load converter; a measuring part that measures the weight in accordance with the electric signal outputted from the first strain sensor; and an estimating part that estimates the active information in accordance with the electric signal outputted from the second strain sensor. In the measuring device, since the weight as a static physical quantity is measured in accordance with the output signal from the first strain sensor excellent in its accuracy and the active information that dynamically changes is estimated in accordance with the output signal from the second strain sensor excellent in its responsiveness, the weight and the active information can be measured with high performance.

The above-described measuring device further comprises a low-pass filter circuit that may change a cut-off frequency in accordance with the electric signal outputted from the second strain sensor. The estimating part may estimate the weight by using the electric signal passing the low-pass filter circuit. According to the measuring device, since the feature of the low-pass filter circuit that filters the output signal of the first strain sensor excellent in its accuracy is dynamically determined by using the output signal of the second strain sensor excellent in its responsiveness, for instance, during a first period immediately after the start of a measuring operation, the weight is measured by using a component of a high frequency to suppress the increase of a time required for measuring the weight, and during a second period subsequent to the first period, the weight is measured by using only a component of a low frequency so that a performance for measuring the load can be improved. Namely, according to this measuring device, the weight can be measured so as to meet an occasion.

Further, in order to solve the above-described problem, the present invention provides a measuring device that measures the weight of a living being and active information showing the living activity of the living being. The measuring device comprises: a plurality of flexure elements that are distorted in accordance with a transmitted load; a third strain sensor that is stuck to one of the plurality of flexure elements to output an electric signal corresponding to the distortion of the one flexure element; a fourth strain sensor that is stuck to the flexure element different from the one flexure element of the plurality of flexure elements to output an electric signal corresponding to the distortion of the different flexure element; a measuring part that measures the weight in accordance with the electric signal outputted from the third strain sensor; and an estimating part that estimates the active information in accordance with the electric signal outputted from the fourth strain sensor and is **characterized in that** the third strain sensor is more excellent in accuracy than the fourth strain sensor and the fourth strain sensor is more excellent in responsiveness than the third strain sensor. In the measuring device, since the weight as a static physical quantity is measured in accordance with the output signal from the third strain sensor excellent in its accuracy and the active information that dynamically changes is estimated in accordance with the output signal from the fourth strain sensor excellent in its responsiveness, the weight and the active information can be measured with high performance. Further, in the measuring device, since the flexure element to which the third strain sensor is stuck is different from the flexure element to which the fourth strain sensor is stuck, a strain sensor (for instance, a strain sensor having pressure sensitive and electrically conductive rubber) may be used that is liable to give an influence to a measuring accuracy of the other strain sensor when one or both of the third strain sensor and the fourth strain sensor is stuck to the same flexure element. That is, this measuring device is advantageously high in degree of freedom of selecting the strain sensor.

According to an aspect of the present invention, there is provided a load converter that converts a load into an electric signal, the load converter having: a flexure element that is distorted in accordance with a load applied to the flexure element; a first strain sensor that is disposed on the flexure element to output a first electric signal corresponding to the distortion of the flexure element; and a second strain sensor that is disposed on the flexure element to output a second electric signal corresponding to the distortion of the flexure element, wherein the first strain sensor is more excellent in accuracy than the second strain sensor, and the second strain sensor is more excellent in responsiveness than the first strain sensor.

According to another aspect of the present invention, there is provided a measuring device that measures the weight of a living being and active information showing the living activity of the living being, the measuring device having: a load converter that converts a load into an electric signal, the load converter having: a flexure element that is distorted in accordance with a load applied to the flexure element; a first strain sensor that is disposed on the flexure element to output a first electric signal corresponding to the distortion of the flexure element; and a second strain sensor that is disposed on the flexure element to output a second electric signal corresponding to the distortion of the flexure element, wherein the first strain sensor is more excellent in accuracy than the second strain sensor, and the second strain sensor is more excellent in responsiveness than the first strain sensor, a measuring part that measures the weight based on the first electric signal; and an estimating part that estimates the active information based on the second electric signal.

According to another aspect of the present invention, there is provided a measuring device that measures the weight of a living being and active information showing the living activity of the living being, the measuring device having: a plurality of flexure elements that are distorted in accordance with a load applied to the flexure elements; a third strain sensor that is disposed on one of the flexure elements to output a third electric signal corresponding to the distortion of the one of the flexure elements; a fourth strain sensor that is disposed on the other one of the flexure element to output a fourth electric signal corresponding to the distortion of the other one of the flexure elements; a measuring part that measures the weight in accordance with the third electric signal; and an estimating part that estimates the active information in accordance with the fourth electric signal, wherein the third strain sensor is more excellent in accuracy than the fourth strain sensor, and the fourth strain sensor is more excellent in responsiveness than the third strain sensor.

According to another aspect of the present invention, there is provided a measuring device that measures the weight of a living being and active information showing the living activity of the living being, the measuring device having: a plurality of load converters that convert a load into an electric signal, each of the load converters having: a flexure element that is distorted in accordance with a load applied to the flexure element; a first strain sensor that is disposed on the flexure element to output a first electric signal corresponding to the distortion of the flexure element; and a second strain sensor that is disposed on the flexure element to output a second electric signal corresponding to the distortion of the flexure element, wherein the first strain sensor is more excellent in accuracy than the second strain sensor, and the second strain sensor is more excellent in responsiveness than the first strain sensor, a measuring part that measures the weight based on the first electric signals output from the load converters; and an estimating part that estimates the active information based on the second electric signals output from the load converters.

### BRIEF DESCRIPTION OF THE SEVERAL VIEWS OF THE DRAWINGS

Fig. 1 is a perspective view showing an external appearance of a bed scale 100 according to one embodiment of the present invention.
Fig. 2 is a diagram showing a using example of the bed scale 100.
Fig. 3A is a longitudinally sectional view showing the structure of a main part of a sensor unit 120 of the bed scale 100.
Fig. 3B is a bottom view showing the structure of a main part of a sensor unit 120 of the bed scale 100.
Fig. 4 is a longitudinally sectional view showing the structure of a main part of a sensor unit 120 of the bed scale 100.
Fig. 5 is a diagram showing an electric structure of a metal strain gauge 123 of the sensor unit 120.
Fig. 6 is a top view showing a structure in the vicinity of the metal strain gauge 123.
Fig. 7 is a block diagram showing the structure of a control unit 110 of the bed scale 100.
Fig. 8 is a perspective view showing an external appearance of a weight scale 200 according to a modified example 1 of the embodiment.
Fig. 9 is a block diagram showing a filter circuit 400 according to a modified example 2 of the embodiment.
Fig. 10 is a diagram for explaining a modified example 4 of the embodiment.

### DETAILED DESCRIPTION

### <Strain sensor>

Now, an embodiment of the present invention will be described below by referring to the drawings. Before the embodiment of the present invention is described, a strain sensor is described. The strain sensor is a sensor for measuring a distortion. The strain sensor includes an electric resistance type that measures the distortion by employing a phenomenon that an electric resistance is changed in accordance with the distortion, a piezoelectric effect type that measures the distortion by employing a piezoelectric effect and an optical type that measures the distortion by employing a phenomenon that an optical variable (for instance, wavelength) is changed in accordance with the distortion.

As the strain sensor of the electric resistance type, a strain gauge or a strain sensor using a pressure sensitive and electrically conductive rubber may be exemplified. The strain gauge is stuck to a flexure element and outputs a signal of a level corresponding to the level of the distortion of the flexure element.

The strain gauge includes a metal strain gauge excellent in its accuracy and a semiconductor strain gauge excellent in its responsiveness. A resistor of the metal strain gauge is made of metal and expanded and contracted in accordance with the distortion of the flexure element. The expansion and contraction change the electric resistance of the resistor. As the metal forming the resistor of the metal strain gauge, platinum or iron, nickel, tungsten, aluminum, gold, copper and silver may be exemplified.

The accuracy of the metal strain gauge is particularly excellent among various kinds of ordinary strain sensors.

The resistor of the semiconductor strain gauge is formed with a semiconductor and the electric resistance of the resistor changes in accordance with the deviation of a crystallographic axis. This deviation arises depending on a stress (including a shearing stress) of the resistor. The stress arises depending on the distortion of the flexure element. As the semiconductor forming the resistor of the semiconductor strain gauge, silicon or germanium may be exemplified. The responsiveness of the semiconductor strain gauge is particularly excellent among various kinds of ordinary strain sensors.

As the strain sensor of the piezoelectric effect type, a piezoelectric element (a piezo-element (including a quartz oscillator)) excellent in its responsiveness may be exemplified. The piezoelectric element has a piezoelectric member and is attached to a flexure element to output a voltage corresponding to the stress (including the shearing stress) of the piezoelectric member. The sensitivity of the piezoelectric element is lower than that of the semiconductor strain gauge. As the strain sensor of the optical type, a strain sensor may be exemplified that employs a BOTDR (Brillouin Optical Time Domain Reflectometer) using Brillouin scattered light obtained when lights are made to be incident on optical fibers or an FBG (Fiber Bragg Grating). In the latter, reflected lights obtained when lights are made to be incident on the FBG are used.

### <Embodiment>

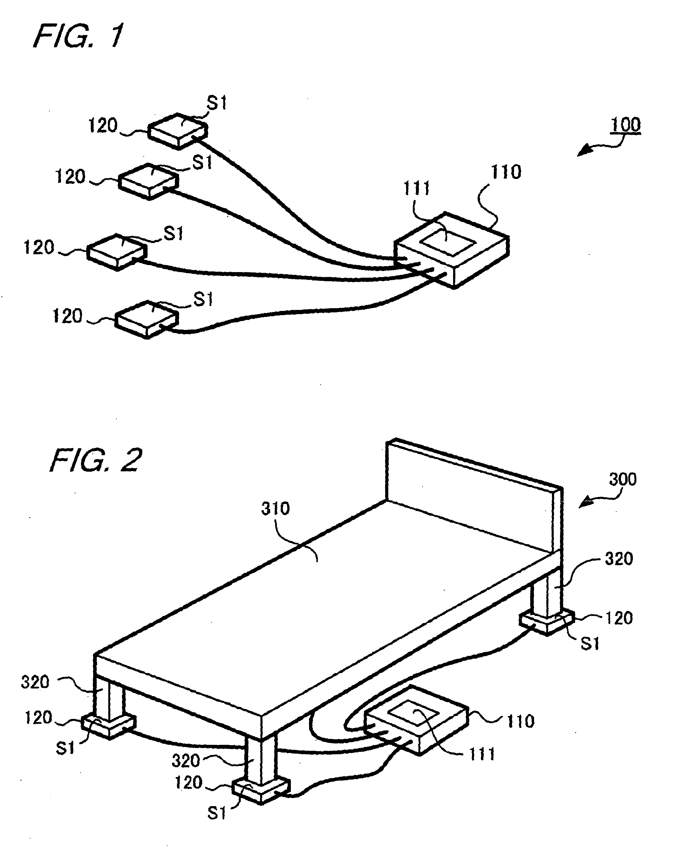

Fig. 1 is a perspective view showing an external appearance of a bed scale 100 according to one embodiment of the present invention. The bed scale 100 measures the weight of a bed on which a living being such as a human being is laid and active information showing the living activity of the living being laid on the bed. As the active information to be measured by the bed scale 100, the number of respirations or the number of heart-beats and a body movement (for instance, a slight movement of an articulation or an oscillation of the center of gravity) may be exemplified.

The bed scale 100 includes a control unit 110 and a plurality of sensor units 120. The sensor units 120 have mount surfaces S1 on which the bed to be measured is mounted to measure an external force (load) that presses the mount surfaces S1 and output a measuring signal showing a measured result. The control unit 110 is electrically connected to the sensor units 120 respectively to control the sensor units 120 respectively and measure the weight and the active information. The number of the sensor units 120 is specifically set to four, because only the bed whose number of legs is four or lower is determined as an object to be measured. That is, the number of the sensor units may be set to a value equal to or more than the number of the legs of the bed to be measured.

Fig. 2 is a diagram showing a using example of the bed scale 100. The bed 300 shown in Fig. 2 includes a main body 310 and four legs 320. The main body 310 is a part in contact with a person who is laid on the bed and is supported by a plurality of legs 320. Between the one leg 320 and a floor, one sensor unit 120 is held. That is, the legs 320 are respectively mounted on the mount surfaces S1 of the corresponding sensor units 120 and the bed 300 is supported by the plurality of the sensor units 120. When the number of the legs of the bed to be measured is smaller than that of the sensor units, the sensor unit exists on which the leg is not mounted. Since a load measured by such a sensor unit is always zero, the sensor unit does not need to be detached.

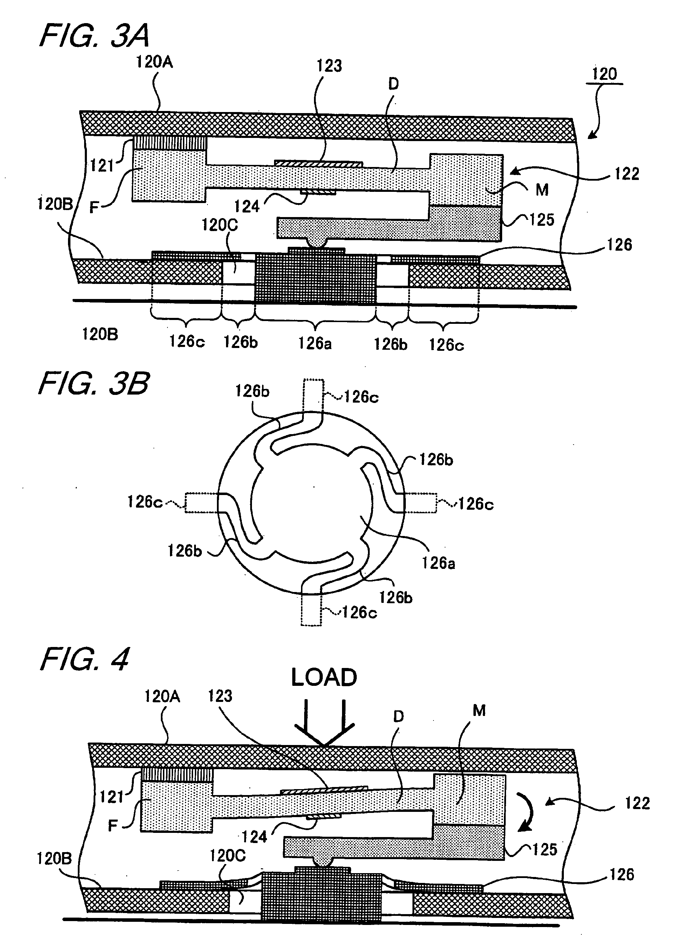

Fig. 3A and Fig. 4 are respectively longitudinally sectional views showing the structure of a main part of the sensor unit 120. Fig. 3B is a bottom view showing the structure of the main part of the sensor unit 120. Fig. 3A shows a state that the load is zero and Fig. 4 shows a state that the load is positive. As shown in these diagrams, the sensor unit 120 includes a fixed part 121 attached to an upper surface 120A, a flexure element 122 distorted in accordance with the load, a metal strain gauge 123, a semiconductor strain gauge 124, a load transmitting part 125 and a leg part 126.

The flexure element 122 is made of metal and includes a fixed end part F, a movable end part M and a distortion part D for connecting both the end parts together. The fixed end part F is attached to the fixed part 121. A bottom surface of the movable end part M is fixed to the load transmitting part 125. The load transmitting part 125 comes into contact with the center of the leg part 126.

The leg part 126 is provided so as to protrude to a floor surface from a hole part 120C formed on a bottom surface 120B of the sensor unit 120. The leg part 126 includes a first member 126a having a cylindrical form, rib parts 126b functioning as elastic members and fixed parts 126c attached to the bottom surface 120B of the sensor unit 120.

The operation of the flexure element 122 and the load transmitting part 125 will be described below. In the state shown in Fig. 3, when the load is exerted on the upper surface 120A of the sensor unit 120, the leg part 126 slides vertically upward to push up the load transmitting part 125. Thus, as shown in Fig. 4, a moment acts on the movable end part M of the flexure element 122 in the direction shown by an arrow mark. As a result, the flexure element 122 is modified in the form of an S shape.

To the upper surface of the distortion part D, the metal strain gauge 123 is attached, and to the lower surface thereof, the semiconductor strain gauge 124 is attached. Both the gauges are arranged at corresponding positions with the distortion part D sandwiched between them and distorted together with the distortion part D. The metal strain gauge 123 and the semiconductor strain gauge 124 output electric signals respectively corresponding to the level of a distortion. Since the level of the distortion is determined in accordance with the level of the load, the flexure element 122, the metal strain gauge 123 and the semiconductor strain gauge 124 function as a load converter for converting the load transmitted by the leg part 126 and the load transmitting part 125 to the electric signal.

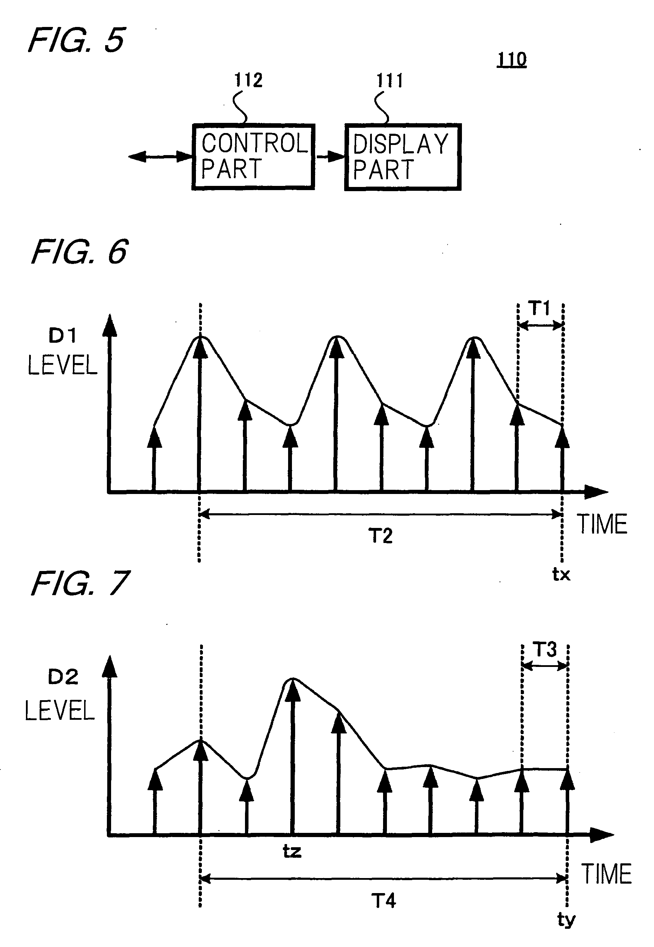

Fig. 5 is a block diagram showing a structure of the control unit 110. The control unit 110 includes a display part 111 and a control part 112. The display part 111 is, for instance, a liquid crystal display. The control part 112 includes a calculating device such as a CPU or a microcomputer to control the supply of an electric power to the metal strain gauge 123 and the semiconductor strain gauge 124 of each sensor unit 120. On the other hand, during a period that the electric power is supplied to the metal strain gauge 123 and the semiconductor strain gauge 124, the control part 112 repeatedly carries out a weight measuring process for measuring the weight and controlling the display part 111 to display a measured value, and repeatedly carries out an active information measuring process for estimating the active information and controlling the display part 111 to display an estimated value.

In the weight measuring process, the control part 112 uses measuring signals outputted from the metal strain gauges 123 of all the sensor units 120 to measure the weight of the bed on which the living being such as the human being is laid. Namely, the control part 112 functions as a measuring part that measures the weight in accordance with the measuring signals outputted from the metal strain gauges 123 of all the sensor units 120. Specifically, the control part 112 carries out a calculation for obtaining the total sum of the load shown by the measuring signals to generate data showing the result of the calculation. Then, the control part 112 uses the generated data and controls the display part 111 to display the calculated result shown by the data, that is, the measured value of the weight of the bed on which the living being such as the human being is laid.

In the active information measuring process, the control part 112 uses measuring signals outputted from the semiconductor strain gauges 124 of all the sensor units 120 to estimate the active information of an object to be measured. Namely, the control part 112 functions as an estimating part that estimates the active information in accordance with the measuring signals outputted from the semiconductor strain gauges 124 of all the sensor units 120. Then, the control part 112 controls the display part 111 to display the estimated active information. The active information is estimated by a prescribed calculating process. As the prescribed calculating process, what calculating process is to be employed may be arbitrarily determined. The prescribed calculating process is different depending on the active information of an object to be measured.

For instance, when the active information of the object to be measured indicates the number of respirations or the number of heart-beats, the control part carries out the calculation for obtaining the total sum of the load shown by the measuring signals outputted from the semiconductor strain gauges 124 of all the sensor units 120 to repeat a first process for generating first data D1 showing the total sum at intervals of first time T1. On the other hand, the control part repeats a second process at intervals of second time T2 in which the first data D1 is used that is generated during a period between a present time and a time earlier by the second time T2 than the present time to estimate the elapsed variation of the total sum and carry out a calculation for estimating the number of respirations or the number of heart-beats on the basis of the elapsed variation.

In an example shown in Fig. 6, the first data D1 is a data series obtained in a sampling period of the first time T1. Assuming that the present time is time tx, the number of heart-beats can be estimated on the basis of nine first data D1 obtained during the period of the second time T2. In order to estimate the number of respirations, the second time T2 may be set to a longer time to extract a lower frequency component included in the first data D1. The second time T2 is longer than the first time T1.

Further, when the active information of the object to be measured indicates the body movement, the control part 112 repeats a third process at intervals of third time T3 in which second data D2 showing the load represented by the measuring signals outputted from the semiconductor strain gauges 124 is generated for each second unit 120. When the four sensor units 120 are used as in this embodiment, the second data D2 is formed by four individual data d1 to d4 corresponding to the sensor units 120 respectively.

Then, the control part 112 carries out a fourth process at intervals of fourth time T4 in which the second data D2 (d1 to d4) is used that is generated during a period between a present time and a time earlier by the fourth time T4 from the present time to estimate the elapsed variation of the load for each sensor unit 120 and carry out a calculation for estimating the slight movement of an articulation or the oscillation of the center of gravity in accordance with the elapsed variation.

In an example shown in Fig. 7, the second data D2 is a data series obtained in a sampling period of the third time T3. Assuming that the present time is time ty, the body movement can be estimated on the basis of nine second data D2 obtained during the period of the fourth time T4. In this example, since the level of the second data D2 is remarkably varied in the vicinity of time tz, the control part can detect that the body movement arises at the time tz. The fourth time T4 is longer than the third time T3.

As described above, the bed scale 100 includes the plurality of the sensor units 120. Each of the sensor units 120 includes the load converter having the flexure element 122 distorted in accordance with the transmitted load, the metal strain gauge 123 attached to the flexure element 122 to output the electric signal corresponding to the distortion of the flexure element 122 and the semiconductor strain gauge 124 attached to the flexure element 122 to output the electric signal corresponding to the distortion of the flexure element 122. Then, in the bed scale 100, the weight is measured in accordance with the output signal of the metal strain gauge 123 excellent in its accuracy and the active information is estimated in accordance with the output signal of the semiconductor strain gauge 124 excellent in its responsiveness. Accordingly, in the bed scale 100, both the weight and the active information can be measured with high performance.

Further, in the bed scale 100, as the strain sensor, are employed the metal strain gauge particularly excellent in its accuracy among ordinary strain sensors and the semiconductor strain sensor particularly excellent in its responsiveness among the ordinary strain sensors. Further, the semiconductor strain gauge has a higher sensitivity than that of other strain sensor (for instance, a piezoelectric element) particularly excellent in its responsiveness among the ordinary strain sensors. Accordingly, the performance of a measurement by the bed scale 100 is extremely high.

### <Modified Examples>

The present invention may include various kinds of modified examples obtained by modifying the above-described embodiment within a scope. These modified examples are partly enumerated below.

### <Modified Example 1>

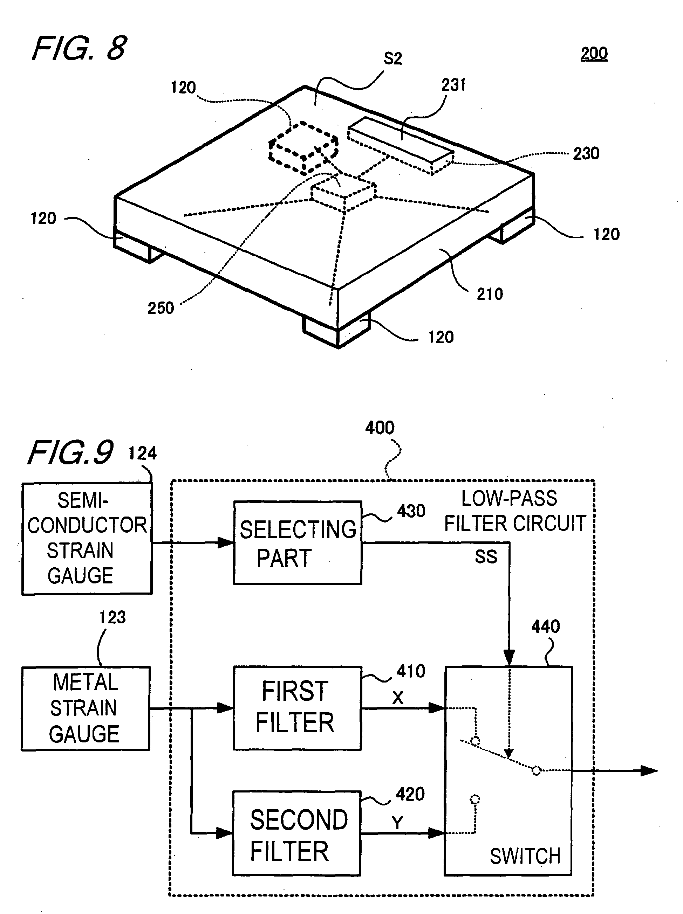

Fig. 8 is a perspective view showing an external appearance of a weight scale 200 obtained by modifying the above-described embodiment. The weight scale 200 is a measuring device that measures the weight of a living being such as a human being and active information showing the living activity of the living being such as the human being. The weight scale 200 includes a main body 210 having a mount surface S2 on which the living being as an object to be measured is mounted and sensor units 120 provided at four corners of the bottom surface of the main body 210. The main body 210 is supported by the four sensor units 120 and slides in a vertical direction in accordance with an external force (a load) that presses the mount surface S2. As the active information to be measured by the weight scale 200, the number of respirations or the number of heart-beats and a body movement (for instance, a slight movement of an articulation or an oscillation of the center of gravity) may be exemplified.

The main body 210 has a display part 230. The display part 230 is, for instance, a liquid crystal display, and includes a display surface 231 forming a part of the mount surface S2 to display information such as the weight or the active information on the display surface 231. The main body 210 has a control part 250 therein.

Measuring signals generated by the sensor units 120 are supplied to the control part 250. The control part 250 estimates the weight and the active information in accordance with the measuring signals and controls the display part 230 to display an estimated value. Namely, the control part 250 functions as a measuring part for measuring the weight in accordance with the measuring signals outputted from metal strain gauges of all the sensor units 120. On the other hand, the control part 250 functions as an estimating part for estimating the active information in accordance with the measuring signals outputted from semiconductor strain gauges of all the sensor units 120. In the weight scale 200, the weight is estimated in accordance with the output signal of the metal strain gauge excellent in its accuracy and the active information is estimated in accordance with the output signal of the semiconductor strain gauge excellent in its responsiveness. Accordingly, the weight scale 200 can obtain the same effect as that of the bed scale 100.

### <Modified Example 2>

The above-described embodiment or the modified example 1 may be modified to provide a low-pass filter in the post-stage of the metal strain gauge for measuring the weight. According to this form, the performance in measuring the weight is improved. However, this form inconveniently requires a long time for measuring the weight. To mitigate the inconvenience, this form may be modified to provide a prescribed low-pass filter circuit in place of the above-described low-pass filter.

Fig. 9 is a block diagram showing a structural example of the prescribed low-pass filter circuit. The low-pass filter circuit 400 of this example includes a first filter 410, a second filter 420, a selecting part 430 and a switch 440. The first filter 410 and the second filter 420 are respectively low-pass filters. A cut-off frequency fc1 of the first filter 410 is higher than a cut-off frequency fc2 of the second filter. Therefore, a first output signal X of the first filter 410 is excellent in its responsiveness, however inferior in its accuracy. A second output signal Y of the second filter 420 is inferior in its responsiveness, however, excellent in its accuracy.

The selecting part 430 inputs an output signal of a semiconductor strain gauge to generate and output a selecting signal SS for selecting either the first output signal X or the second output signal Y in accordance with the output signal. More specific contents of the selecting signal SS are arbitrary. In this example, when the level of the output signal of the semiconductor strain gauge shifts from a level showing a load of zero to a level showing a positive load, the selecting signal SS becomes a low level only during a fixed period (a first period) and becomes a high level during a second period subsequent to the first period. When the level of the output signal of the semiconductor strain gauge shifts from the level corresponding to the positive load to the level corresponding to zero of the load, the second period is finished.

The switch 440 outputs one of the first output signal X and the second output signal Y in accordance with the selecting signal SS. Specifically, the switch 440 outputs the first output signal X when the selecting signal SS is in the low level, and outputs the second output signal Y when the selecting signal is in the high level.

After all, the low-pass filter circuit 400 serves to filter the output signal of a metal strain gauge excellent in its accuracy and selects the cut-off frequency by using the output signal of the semiconductor strain gauge excellent in its responsiveness. That is, the feature of the low-pass filter circuit 400 is dynamically determined by using the output signal of the semiconductor strain gauge excellent in its responsiveness.

As described above, since the second filter 420 is inferior in responsiveness to the first filter 410, even when the load is applied to the weight scale, the second output signal Y rises gently. As compared therewith, the first output signal X steeply rises. Accordingly, during a prescribed period after the load is applied, since the second output signal does not sufficiently rise, the first output signal X is rather higher in accuracy than the second output signal Y. On the other hand, when a certain time elapses, the second output signal Y is higher in accuracy than the first output signal X.

Thus, the selecting signal SS is used to select the first output signal X during the first period and select the second output signal Y during the second period, so that the responsiveness is improved and the accuracy of a measurement is improved in a steady state.

The first filter 410 and the second filter 420 may be formed with a program for calculating a moving average. In this case, the number of samples used as objects to calculate the moving average in the first filter 410 is smaller than the number of samples used as objects to calculate the moving object in the second filter 420.

Further, as another structural example of the prescribed low-pass filter circuit, a structure may be exemplified that employs a low-pass filter capable of changing a cut-off frequency in place of the first filter 410, the second filter 420 and the switch 440. In this structure, when a selecting signal SS from a selecting part 430 is supplied to the low-pass filter, the cut-off frequency (fc1 or fc2) is set in accordance with the selecting signal SS to carry out a filtering process.

### <Modified Example 3>

The above-described embodiment or the modified example 1 may be modified to provide a device including a load converter having no metal strain gauge or a load converter having no semiconductor strain gauge. Namely, the device may include a plurality of flexure elements that are distorted in accordance with a transmitted load; a metal strain gauge (a third strain sensor) that is stuck to one of the plurality of flexure elements to output an electric signal corresponding to the distortion of the one flexure element; a semiconductor strain gauge (a fourth strain sensor) that is stuck to the flexure element different from the flexure element of the plurality of flexure elements to which the metal strain gauge is stuck to output an electric signal corresponding to the distortion of the different flexure element; a measuring part that measures a weight in accordance with the electric signal outputted from the third strain sensor; and an estimating part that estimates active information in accordance with the electric signal outputted from the fourth strain sensor.

### <Modified Example 4>

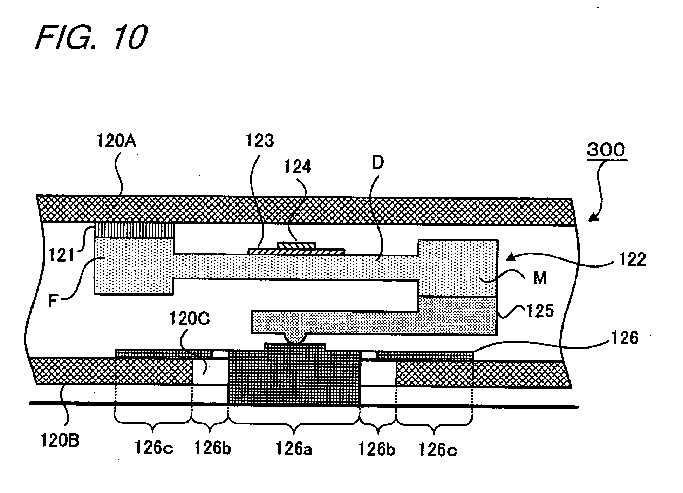

In the above-described embodiment or the modified examples respectively, the metal strain gauge is attached to the upper surface of the distortion part and the semiconductor strain gauge is attached to the lower surface, however, the present invention is not limited thereto. The semiconductor strain gauge may be attached to the upper surface of the distortion part and the metal strain gauge may be attached to the lower surface. Further, in the above-described embodiment or the modified examples respectively, all the strain gauges come into contact with the flexure element, however, the present invention is not limited thereto. A strain gauge may not come into contact with a flexure element. One example showing the above-described state is illustrated in Fig. 10. In a sensor unit 300 of this example, a semiconductor strain gauge 124 comes into contact with a metal strain gauge 123 and is attached to a distortion part D through the metal strain gauge 123.

### <Modified Example 5>

In the above-described embodiment and the modified examples respectively, as the combination of the strain sensors, the combination of the metal strain gauge and the semiconductor strain gauge is employed. However, the present invention is not limited thereto. That is, when a combination satisfies a condition that one strain sensor is more excellent in responsiveness than the other strain sensor and the other strain sensor is more excellent in accuracy that one strain sensor, arbitrary strain sensors may be employed.

### <Modified Example 6>

In the above-described embodiment or the modified examples respectively, as the active information to be measured, the number of respirations or the number of heart-beats and the body movement are exemplified, and, other active information than them may be set as an object to be measured. For instance, in the case of the bed scale, sleeping hours, a time required for a person to go to bed and then become drowsy, the number of times of awakening during a sleeping period may be set as objects to be measured. Further, the soundness, the quality and the efficiency of sleeping may be set as objects to be measured. The above-described active information is obtained by a statistical estimation based on, for instance, the number of respirations or the number of heart-beats and the body movement.

## Claims

1. A load converter that converts a load into an electric signal, the load converter comprising:
a flexure element that is distorted in accordance with a load applied to the flexure element;
a first strain sensor that is disposed on the flexure element to output a first electric signal corresponding to the distortion of the flexure element; and
a second strain sensor that is disposed on the flexure element to output a second electric signal corresponding to the distortion of the flexure element,
wherein the first strain sensor is more excellent in accuracy than the second strain sensor, and
the second strain sensor is more excellent in responsiveness than the first strain sensor.

2. The load converter according to claim 1, wherein the first strain sensor is a metal strain gauge and the second strain sensor is a semiconductor strain gauge.

3. A measuring device that measures the weight of a living being and active information showing the living activity of the living being, the measuring device comprising:
the load converter according to claim 1 or 2;
a measuring part that measures the weight based on the first electric signal; and
an estimating part that estimates the active information based on the second electric signal.

4. The measuring device according to claim 3, further comprising a low-pass filter circuit that is configured to be changed a cut-off frequency of the low-pass filter circuit based on the second electric signal, and
wherein the measuring part measures the weight by using the first electric signal passing through the low-pass filter circuit.

5. A measuring device that measures the weight of a living being and active information showing the living activity of the living being, the measuring device comprising:
a plurality of flexure elements that are distorted in accordance with a load applied to the flexure elements;
a third strain sensor that is disposed on one of the flexure elements to output a third electric signal corresponding to the distortion of the one of the flexure elements;
a fourth strain sensor that is disposed on the other one of the flexure element to output a fourth electric signal corresponding to the distortion of the other one of the flexure elements;
a measuring part that measures the weight in accordance with the third electric signal; and
an estimating part that estimates the active information in accordance with the fourth electric signal,
wherein the third strain sensor is more excellent in accuracy than the fourth strain sensor, and
the fourth strain sensor is more excellent in responsiveness than the third strain sensor.

6. A measuring device that measures the weight of a living being and active information showing the living activity of the living being, the measuring device comprising:
a plurality of load converters according to claim 1 or 2;
a measuring part that measures the weight based on the first electric signals output from the load converters; and
an estimating part that estimates the active information based on the second electric signals output from the load converters.

7. The load converter according to claim 1 or 2 , wherein the flexure element has a first face and a second face that is formed opposite face to the first face,
the first strain sensor is disposed on the first face, and
the second strain sensor is disposed on the second face.

8. The load converter according to any of claims 1, 2 and 7 wherein the second strain sensor that is disposed on the flexure element via the first strain sensor, so that the first strain sensor is disposed between the second strain sensor and the flexure element.

9. The measuring device according to claim 3, wherein the active information includes at least one of the number of respirations, the number of heart-beats, and a body movement of the living being.

10. The measuring device according to claim 3, wherein the active information includes the number of respirations and the number of heart-beats,
the estimating part estimates the number of respirations from first data that is obtained by sampling the second electric signal at a first sampling period, and
the estimating part estimates the number of heart-beats from second data that is obtained by sampling the second electric signal at a second sampling period that is longer than the first sampling period.

11. The measuring device according to any of claims 3, 5, 6, 9 and 10 further comprising a low-pass filter circuit including:
a first low-pass filter having a first cut-off frequency;
a second low-pass filter having a second cut-off frequency higher than the first cut-off frequency; and
a selector that is configured to selectively output the first electric signal passing through the first low-pass filter or the first electric signal passing through the second low-pass filter, and
wherein the measuring part measures the weight by using the electric signal output from the selector.

12. The measuring device according to claim 6, wherein the active information includes at least one of the number of respirations, the number of heart-beats, and a body movement of the living being.

13. The measuring device according to claim 6, wherein the active information includes the number of respirations, the number of heart-beats, and a body movement of the living being,
the estimating part estimates the number of respirations from first data that is obtained by sampling the second electric signal at a first sampling period,
the estimating part estimates the number of heart-beats from second data that is obtained by sampling the second electric signal at a second sampling period that is longer than the first sampling period, and
the estimating part estimates the body movement from third data that is obtained by sampling the second electric signal at a third sampling period that is longer than the second sampling period.
